# EUROPÄISCHE PATENTSCHRIFT

(11) **EP 0 698 591 B1**
(45) Veröffentlichungstag und Bekanntmachung des Hinweises auf die Patenterteilung: **25.09.2002**
(21) Anmeldenummer: 95109603.1
(22) Anmeldetag: 21.06.1995
(51) Int. Cl.: C07C 37/14, C08G 59/24, C08G 59/32

(54) **Alkylierte und aralkylierte Polyhydroxyaromaten und ein Verfahren zu ihrer Herstellung**
Alkylated and aralkylated polyhydroxy aromatic compounds and a process for their preparation
Composés polyhydroxyles aromatiques alcoylés ou aralcoylés et un procédé pour leur préparation

(30) Priorität: 30.06.1994 DE 4422869; 10.10.1994 DE 4436097
(43) Veröffentlichungstag der Anmeldung: 28.02.1996
(73) Patentinhaber: Solutia Germany GmbH & Co. KG, 55252 Mainz-Kastel (DE)
(72) Erfinder: Neumann, Uwe, Dr., D-65307 Bad Schwalbach (DE)

(56) Entgegenhaltungen:
- DE-A- 1 543 512
- US-A- 5 300 618

## Beschreibung

Die Erfindung betrifft ein Verfahren zur Herstellung von alkylierten und aralkylierten Polyhydroxyaromaten.

Polyhydroxyaromaten werden vor allem in der Polymerchemie als Edukte zur Herstellung von Polycarbonaten, Polyestern, Polyäthern und Epoxidharzen eingesetzt. Unter Polyhydroxyaromaten werden im Folgenden aromatische Verbindungen mit zwei oder mehr Hydroxylgruppen im Molekül verstanden. Die aromatischen Verbindungen sind ein- oder mehrkernig, sie können als Strukturelemente auch beliebige Kombinationen von einzelnen und anellierten Ringen, und zweibindigen Gruppen wie (Thio-)-Ätherbrücken, Carbonylgruppen, Sulfongruppen, Carbonamidgruppen und Alkylengruppen enthalten.

Während beispielsweise die vom Bisphenol A abgeleiteten Polycarbonate und Polyphthalate gut in aromatischen Lösungsmitteln löslich sind, lösen sich die in der Technik üblichen Epoxidharze nicht oder nur in geringem Maß in aromatischen Lösungsmitteln. Die als Lösungsmittel geeigneten aliphatischen Äther (wie z.B Methoxypropanol, Äthylenglykol-monobutyläther, Diäthylenglykoldimethyläther), sind einerseits erheblich teurer, andererseits neigen sie im Kontakt mit Luftsauerstoff zur Bildung von Peroxiden und sind daher in der Handhabung aufwendiger und bedeuten ein Sicherheitsrisiko.

Daher bestand die Aufgabe, die bekannten Polyhydroxyaromaten so zu modifizieren, daß die Löslichkeit der daraus hergestellten Polykondensate in aromatischen Lösungsmitteln verbessert wird. Dabei sollte das ansonsten gute Eigenschaftsniveau der daraus hergestellten Polymere erhalten bleiben.

Es gab bereits Versuche, durch Substitution von Phenolen mit Arylresten oder Aralkylresten zu modifizierten Phenolen zu gelangen. So ist in DE-A 19 40 220 ein Verfahren zur Herstellung von Aralkylphenolen beschrieben durch Umsetzung einer aromatischen Vinylidenverbindung mit einem Phenol unter Katalyse mit Säuren oder Friedel-Crafts-Katalysatoren. Gemäß den Ausführungen in der österreichischen Patentschrift AT 284 444 sind die Reaktionen von (gegebenenfalls substituierten) Styrolen mit Phenolen bekannt. Bei diesen Reaktionen wird die Vinylgruppe der Styrole in ortho- oder para-Stellung zur OH-Gruppe des Phenols addiert. Zur Beschleunigung werden im allgemeinen Friedel-Crafts-Katalysatoren, z.B. Säuren und Metallhalogenide, verwendet. In Abhängigkeit von den Reaktionsbedingungen, Katalysatoren und Mengenverhältnissen der Reaktionsteilnehmer erhält man dabei mono-, di- oder tristyrolisierte Phenole (loc. cit. Seite 1, Zeilen 23 bis 29). Unter den Bedingungen der Friedel-Crafts-Reaktion finden jedoch auch Isomerisierungsreaktionen beispielsweise bei den Bis- oder Poly(hydroxyphenyl)alkanen statt, da die Bindung zwischen Aromat und Alkylengruppe unter diesen Bedingungen auch gelöst wird. Man erhält Mischungen von unterschiedlichst substituierten Polyhydroxyaromaten, zum Teil werden auch Phenol oder andere leicht flüchtige Hydroxyaromaten abgespalten. Je nach Temperatur, Reaktionszeit und Umsetzungsbedingungen erhält man hochvernetzte spröde Produkte oder gummiartige, klebrige Produkte, wenn die so erhaltenen Hydroxyaromaten mit Epichlorhydrin oder mit Diglycidylverbindungen zu Epoxidharzen umgesetzt werden.

Zur Modifizierung von Phenolen als Ausgangsverbindungen für Epoxid-Grundkörper sind Methoden zur Kern-Alkylierung beschrieben, so z.B. mit Olefinen (K.-D. Bode in Houben-Weyl: Methoden der Organischen Chemie, 4.Aufl., Bd. 6/1c, S.955ff., Georg Thieme Verlag, Stuttgart 1976). Die Herstellung von Epoxiden daraus gelingt z.B. wie in US-A 4,594,398 aufgeführt, indem nach kationischer Alkylierung von Phenol mit aliphatischen Dienen in Gegenwart von Katalysatoren mit Epichlorhydrin zum Diglycidyläther umgesetzt und so Bisepoxide erhalten werden. Durch diese Substitutionsreaktionen werden zusätzlich Alkylgruppen eingeführt, dabei wird jedoch die Löslichkeit in aromatischen Kohlenwasserstoffen nicht verbessert. Die Reaktionsprodukte sind keine einheitlichen Körper, sondern Gemische von verschieden substituierten Polyhydroxyaromaten mit unterschiedlicher Anzahl von Hydroxylgruppen im Molekül. In der DE-A 1 543 512 wird ein Verfahren zur Aralkylierung von Phenolen mit 1-Aralk-2-enen beschrieben, wobei Oxalsäure als Katalysator eingesetzt wird. Es entstehen Gemische unterschiedlich alkylierter Phenole. In der US-A 5,300,618 wird ebenfalls unter anderem Oxalsäure als Katalysator bei der Aralkylierung von Resorcin eingesetzt.

Die Aufgabe der Erfindung ist daher, ein Verfahren zur Alkylierung und Aralkylierung zu finden, das für alle der oben genannten Polyhydroxyaromaten anwendbar ist, und das zu einheitlichen Produkten ohne Umlagerung und ohne Abspaltung von Phenol(en) führt.

Erfindungsgemäß wurde diese Aufgabe gelöst, indem man zu einer Mischung der Polyhydroxyaromaten-Komponente und der Alkylenoder Alkenylaromaten-Komponente eine Mischung von Oxalsäure und Borsäure im Molverhältnis von 1:5 bis 1:0 zufügt, wobei der Massenanteil der Säuremischung in der gesamten Reaktionsmischung bevorzugt zwischen 0,5 und 10 % beträgt.

In den der Erfindung zugrundeliegenden Untersuchungen ließ sich zeigen, daß Ameisensäure, Essigsäure und höhere Carbonsäuren allein oder in Mischung mit Borsäure keine katalytische Aktivität für die Alkylierungs- und Aralkylierungsreaktion besitzen.

Gegenstand der Erfindung ist ein Verfahren zur Herstellung von alkylierten und aralkylierten Polyhydroxyaromaten durch Umsetzen von einem Polyhydroxyaromat (A) mit einer Alkylenverbindung (B) bei erhöhter Temperatur unter Zusatz einer Mischung (C) von Oxalsäure und Borsäure im Molverhältnis von 1:5 bis 1:0,1, vorzugsweise 1:2 bis 1:0,1, insbesondere 1:1 bis 1:0,5. Die Reaktion kann in der Schmelze (Masseverfahren) oder in einem inerten hochsiedenden Lösungsmittel durchgeführt werden.

Die Reaktion wird bei einer Temperatur von 80 bis 180 °C, bevorzugt von 100 bis 170 °C und besonders bevorzugt von 120 bis 160 °C durchgeführt.

Zur Vermeidung von oxydativer Schädigung der Edukte und Produkte wird die Reaktion bevorzugt unter Schutzgas (z.B. Stickstoff, Argon) durchgeführt. Falls niedrigsiedende Edukte eingesetzt werden, kann auch unter Druck gearbeitet werden.

Für die Erfindung geeignete Polyhydroxyaromaten sind besonders Dihydroxyaromaten und Trihydroxyaromaten, jeweils einzeln oder im Gemisch.

Geeignete Dihydroxyaromaten sind beispielsweise die Bis(4-hydroxyphenyl)(cyclo)alkane wie das 2,2-Bis-(4-hydroxyphenyl)-propan, Bis(4-hydroxyphenyl)methan und -äthan, 1,1-Bis-(4-hydroxyphenyl)cyclohexan, die entsprechenden vom Naphthol abgeleiteten Verbindungen, höhere Polyhydroxyaromaten wie das 1,4-Bis-(4'-hydroxycumyl)benzol, die Dihydroxy-Benzol- und -Naphthalin-Isomeren, Dihydroxydiphenyl, Dihydroxydiphenyläther, Dihydroxybenzophenon, Dihydroxydiphenylsulfid und -sulfon. Diese Polyhydroxyaromaten können einzeln oder im Gemisch eingesetzt werden.

Für die Erfindung geeignete Alkylen-Verbindungen sind beispielsweise lineare, cyclische und verzweigte Olefine mit vier bis sechzehn Kohlenstoffatomen wie 1-Buten, 2-Buten, 1-Hexen, 1-Octen, 2-Äthyl-hexen-1, 1-Decen, 1-Dodecen, Diolefine wie Butadien-1,3, Hexadien-1,3, Hexadien-1,5, Norbornen, Isopren, und cyclische Diolefine wie Cyclopentadien und Dicyclopentadien. Diese Verbindungen können einzeln oder im Gemisch eingesetzt werden. Bei der Verwendung niedriger Olefine wie Buten ist es vorteilhaft, Mischungen solcher Olefine mit Styrol oder anderen Alkenylaromaten einzusetzen.

Für die Erfindung geeignete Alkenylaromaten sind beispielsweise Styrol und dessen Homologe wie α-Methylstyrol und die isomeren Vinyltoluole sowie deren technische Isomerenmischung, die isomeren Äthylstyrole, Inden, sowie halogenierte Styrole wie Mono- und Di-Chlorstyrol. Diese Verbindungen können einzeln oder im Gemisch eingesetzt werden.

Die alkylierten und aralkylierten Verbindungen gemäß der Erfindung lassen sich als Polyhydroxykomponente in Kondensationspolymeren wie beispielsweise Polyestern, Polycarbonaten, aromatischen Polyäthern und Epoxidharzen an Stelle oder in Mischung mit den üblichen Polyhydroxyverbindungen verwenden. Durch die Substitution werden charakteristische Eigenschaften der erhaltenen Polymere wie beispielsweise Löslichkeit, Glasübergangstemperatur, Kristallinität beeinflußt. Dabei reichen häufig geringe Beimengungen der erfindungsgemäßen Polyhydroxyaromaten zu den üblicherweise verwendeten aus, um deutliche Verschiebungen des Eigenschaftsbildes zu erreichen.

In den nachfolgenden Beispielen wird die Erfindung näher beschrieben.

### Beispiele

### Beispiel 1:

200 g Bisphenol F werden unter Stickstoff auf 120 °C aufgeheizt. 2,2 g Oxalsäure-dihydrat und 1,2 g Borsäure werden zugesetzt. Insgesamt 104 g Styrol werden durch einen Tropftrichter langsam so zugetropft, daß die Temperatur 160 °C nicht überschreitet. Bei 160 °C wird weitere zwei Stunden gerührt und danach abgekühlt. Das Reaktionsprodukt enthält nach Analyse im Mittel eine Phenyläthylgruppe pro Bisphenol F-Molekül.

### Beispiel 2:

Unter Stickstoff werden 228 g Bisphenol A auf 160 °C erwärmt. 4,2 g Oxalsäure-Dihydrat und 1 g Borsäure werden zugefügt. Insgesamt 208 g Styrol werden so zugetropft, daß die Temperatur des Reaktionsgutes zwischen 155 und 165 °C gehalten wird. Nach beendeter Zugabe wird noch zwei Stunden bei 160 °C weiter gerührt. Das Reaktionsprodukt enthält nach Analyse im Mittel zwei Phenyläthylgruppen pro Bisphenol A-Molekül.

### Beispiel 3:

Unter Stickstoff werden 110 g Resorcin auf 120 °C erwärmt. 2,2 g Oxalsäure-Dihydrat und 0,5 g Borsäure werden zugefügt. Insgesamt 130 g Styrol werden so zugetropft, daß die Temperatur des Reaktionsgutes zwischen 120 und 130 °C gehalten wird. Nach beendeter Zugabe wird noch zwei Stunden bei 160 °C weiter gerührt. Das Reaktionsprodukt enthält nach Analyse im Mittel 1,25 Phenyläthylgruppen pro Resorcin-Molekül.

### Beispiel 4:

Unter Stickstoff werden 114 g Bisphenol A auf 160 °C erwärmt. 1 g Oxalsäure-Dihydrat und 0,8 g Borsäure werden zugefügt. Insgesamt 52 g Styrol werden so zugetropft, daß die Temperatur des Reaktionsgutes zwischen 155 und 165 °C gehalten wird. Nach beendeter Zugabe wird noch zwei Stunden bei 160 °C weiter gerührt. Das Reaktionsprodukt enthält nach Analyse im Mittel eine Phenyläthylgruppe pro Bisphenol A-Molekül.

### Beispiel 5 (Vergleichsbeispiel):

Unter Stickstoff werden 114 g Bisphenol A auf 160 °C erwärmt. 2 g wasserfreies Aluminiumchlorid werden zugefügt. Insgesamt 52 g Styrol werden so zugetropft, daß die Temperatur des Reaktionsgutes zwischen 155 und 165 °C gehalten wird. Dabei macht sich ein Geruch nach Phenol bemerkbar. Nach beendeter Zugabe wird noch zwei Stunden bei 160 °C weiter gerührt. Man erhält ein braun verfärbtes Produkt mit uneinheitlicher Zusammensetzung.

## Patentansprüche

1. Verfahren zur Herstellung von alkylierten und von aralkylierten Polyhydroxyaromaten durch Umsetzen von einem Polyhydroxyaromat (A) mit einer Alkylenverbindung (B) bei erhöhter Temperatur in Gegenwart einer Mischung (C) von Oxalsäure und Borsäure im Molverhältnis von 1:5 bis 1:0,1.

2. Verfahren zur Herstellung von alkylierten und aralkylierten Polyhydroxyaromaten gemäß Anspruch 1, **dadurch gekennzeichnet, daß** die Reaktion in der Schmelze (Masseverfahren) durchgeführt wird.

3. Verfahren zur Herstellung von alkylierten und aralkylierten Polyhydroxyaromaten gemäß Anspruch 1, **dadurch gekennzeichnet, daß** die Reaktion in einem inerten hochsiedenden Lösungsmittel durchgeführt wird.

4. Verfahren zur Herstellung von alkylierten und aralkylierten Polyhydroxyaromaten gemäß Anspruch 1, **dadurch gekennzeichnet, daß** die Reaktion bei einer Temperatur von 80 bis 180 °C durchgeführt wird.

5. Verfahren zur Herstellung von alkylierten und aralkylierten Polyhydroxyaromaten gemäß Anspruch 1, **dadurch gekennzeichnet, daß** als Komponente (A) Dihydroxyaromaten und Trihydroxyaromaten jeweils einzeln oder im Gemisch eingesetzt werden.

6. Verfahren zur Herstellung von alkylierten und aralkylierten Polyhydroxyaromaten gemäß Anspruch 1, **dadurch gekennzeichnet, daß** als Komponente (A) Dihydroxyaromaten ausgewählt aus der Gruppe Bisphenol A, Bisphenol F, Hydrochinon, Resorcin und Dihydroxynaphthalin eingesetzt werden.

7. Verfahren zur Herstellung von alkylierten und aralkylierten Polyhydroxyaromaten gemäß Anspruch 1, **dadurch gekennzeichnet, daß** als Komponente (B) Alkenylaromaten, Diolefine oder Mischungen dieser Verbindungen eingesetzt werden.

8. Verfahren zur Herstellung von alkylierten und aralkylierten Polyhydroxyaromaten gemäß Anspruch 1, **dadurch gekennzeichnet, daß** als Komponente (B) Styrol oder Vinyltoluol eingesetzt wird.

9. Verfahren zur Herstellung von alkylierten und aralkylierten Polyhydroxyaromaten gemäß Anspruch 1, **dadurch gekennzeichnet, daß** als Komponente (C) ein Gemisch aus Oxalsäure und Borsäure in einer Menge von 0,5 bis 10 % eingesetzt wird, bezogen auf die Masse der Reaktionsmischung.

## Claims

1. A process for the preparation of alkylated and of aralkylated polyhydroxy aromatic compounds by reaction of a polyhydroxy aromatic compound (A) with an alkylene compound (B) at elevated temperature in the presence of a mixture (C) of oxalic acid and boric acid in a molar ratio of from 1:5 to 1:0.1.

2. The process for the preparation of alkylated and aralkylated polyhydroxy aromatic compounds as claimed in claim 1, wherein the reaction is carried out in the melt (bulk process).

3. The process for the preparation of alkylated and aralkylated polyhydroxy aromatic compounds as claimed in claim 1, wherein the reaction is carried out in an inert, high-boiling solvent.

4. The process for the preparation of alkylated and aralkylated polyhydroxy aromatic compounds as claimed in claim 1, wherein the reaction is carried out at a temperature of from 80 to 180°C.

5. The process for the preparation of alkylated and aralkylated polyhydroxy aromatic compounds as claimed in claim 1, wherein dihydroxy and trihydroxy aromatic compounds are employed as component (A), in each case individually or in a mixture.

6. The process for the preparation of alkylated and aralkylated polyhydroxy aromatic compounds as claimed in claim 1, wherein dihydroxy aromatic compounds selected from the group consisting of bisphenol A, bisphenol F, hydroquinone, resorcinol and dihydroxynaphthalene are employed as component (A).

7. The process for the preparation of alkylated and aralkylated polyhydroxy aromatic compounds as claimed in claim 1, wherein alkenylaromatic compounds, diolefins or mixtures of these compounds are employed as component (B).

8. The process for the preparation of alkylated and aralkylated polyhydroxy aromatic compounds as claimed in claim 1, wherein styrene or vinyltoluene is employed as component (B).

9. The process for the preparation of alkylated and aralkylated polyhydroxy aromatic compounds as claimed in claim 1, wherein a mixture of oxalic acid and boric acid in a quantity of from 0.5 to 10 %, based on the mass of the reaction mixture, is employed as component (C).

## Revendications

1. Procédé pour la préparation de composés polyhydroxyaromatiques alkylés et aralkylés par réaction d'un composé polyhydroxyaromatique (A) avec un composé alkylène (B) à température élevée en présence d'un mélange (C) d'acide oxalique et d'acide borique dans le rapport molaire de 1 : 5 à 1 : 0,1.

2. Procédé pour la préparation de composés polyhydroxyaromatiques alkylés et aralkylés selon la revendication 1, **caractérisé en ce que** la réaction est . réalisée à l'état fondu (procédé en masse).

3. Procédé pour la préparation de composés polyhydroxyaromatiques alkylés et aralkylés selon la revendication 1, **caractérisé en ce que** la réaction est réalisée dans un solvant inerte à point d'ébullition élevé.

4. Procédé pour la préparation de composés polyhydroxyaromatiques alkylés et aralkylés selon la revendication 1, **caractérisé en ce que** la réaction est réalisée à une température de 80 à 180°C.

5. Procédé pour la préparation de composés polyhydroxyaromatiques alkylés et aralkylés selon la revendication 1, **caractérisé en ce qu'**on utilise comme composant (A) des composés dihydroxyaromatiques et trihydroxyaromatiques respectivement seuls ou en mélange.

6. Procédé pour la préparation de composés polyhydroxyaromatiques alkylés et aralkylés selon la revendication 1, **caractérisé en ce qu'**on utilise comme composant (A) des composés dihydroxyaromatiques choisis parmi le bisphénol A, le bisphénol F, l'hydroquinone, la résorcine et le dihydroxynapthalène.

7. Procédé pour la préparation de composés polyhydroxyaromatiques alkylés et aralkylés selon la revendication 1, **caractérisé en ce qu'**on utilise comme composant (B) des composés alcényle aromatiques, des dioléfines ou des mélanges de ces composés.

8. Procédé pour la préparation de composés polyhydroxyaromatiques alkylés et aralkylés selon la revendication 1, **caractérisé en ce qu'**on utilise comme composant (B) le styrène ou le vinyltoluène.

9. Procédé pour la préparation de composés polyhydroxyaromatiques alkylés et aralkylés selon la revendication 1, **caractérisé en ce qu'**on utilise comme composant (C) un mélange d'acide oxalique et d'acide borique en une quantité de 0,5 à 10 %, par rapport à la masse du mélange réactionnel.
